(19) Europäisches Patentamt
European Patent Office
Office européen des brevets

(11) **EP 0 823 442 B1**

(12) **EUROPÄISCHE PATENTSCHRIFT**

(45) Veröffentlichungstag und Bekanntmachung des Hinweises auf die Patenterteilung:
**02.05.2003 Patentblatt 2003/18**

(51) Int Cl.[7]: **C08F 246/00**, C07C 255/50, C07C 255/54, C09K 19/38, G11B 7/24

(21) Anmeldenummer: **97112809.5**

(22) Anmeldetag: **25.07.1997**

(54) **Photoadressierbare Seitengruppenpolymere**

Polymers with photosensitive side chains

Polymères avec des chaînes latérales photosensitives

(84) Benannte Vertragsstaaten:
**DE FR GB IT NL SE**

(30) Priorität: **07.08.1996 DE 19631864**

(43) Veröffentlichungstag der Anmeldung:
**11.02.1998 Patentblatt 1998/07**

(73) Patentinhaber: **BAYER AG**
**51368 Leverkusen (DE)**

(72) Erfinder:
- **Berneth, Horst, Dr.**
  **51373 Leverkusen (DE)**
- **Claussen, Uwe, Dr.**
  **51379 Leverkusen (DE)**
- **Kostromine, Serguei, Dr.**
  **53913 Swisttal (DE)**
- **Neigl, Ralf, Dr.**
  **51373 Leverkusen (DE)**
- **Ruebner, Joachim, Dr.**
  **10409 Berlin (DE)**
- **Ruhmann, Ralf, Dr.**
  **12619 Berlin (DE)**

(56) Entgegenhaltungen:
**EP-A- 0 090 282**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft photoadressierbare Seitengruppenpolymere, in denen sich durch Bestrahlung eine hohe Doppelbrechung induzieren läßt, so daß sie sich zur Herstellung von Bauelementen für die Speicherung optisch angebotener Information oder als optisch schaltbare Komponenten eignen.

Als photoadressierbare Seitengruppenpolymere hat man spezielle verzweigte Polymere empfohlen: An einem linearen Rückgrat sitzen - über als Abstandshalter wirkende Molekülteile verbunden - Seitengruppen unterschiedlicher Art, von denen eine Art ("A" genannt) elektromagnetische Strahlung absorbieren kann und eine andere Art ("M" genannt) eine mesogene, formanisotrope Gruppe darstellt.

Um die Wechselwirkung der mesogenen Gruppen nicht zu behindern, hat man die mesogenen Gruppen in der Vergangenheit meist über ein Sauerstoffatom an die abstandshaltende Gruppe gekoppelt, weil man bisher angenommen hat, daß höherwertige Atome mit ihren Substituenten an dieser Stelle aufgrund ihres Raumbedarfs die Wechselwirkung und damit die Photoadressierbarkeit behindern.

Der Mechanismus der photoadressierten Ausrichtung beruht wahrscheinlich auf der Möglichkeit, durch elektromagnetische Strahlung eine Ausrichtung der mesogenen Gruppen und damit eine Änderung des Ordnungszustands zu erreichen. Die bislang bekannten photoadressierbaren Polymeren sind allerdings noch mit dem Nachteil behaftet, daß die erzeugbaren Änderungen des Ordnungszustands zu gering sind, die Änderung des Ordnungszustands zu langsam erfolgt und/oder die eingeschriebenen Muster beim Lagern langsam wieder verblassen.

Aufgabe der Erfindung war es daher, Polymere bereitzustellen, die diese Nachteile nicht oder in geringerem Ausmaß besitzen.

Überraschenderweise wurde nun ein Zusammenhang zwischen der experimentell bestimmbaren Wechselwirkung der Gruppe A, die elektromagnetische Strahlung absorbieren kann, gegenüber einem Standard und der Qualität der Photoadressierbarkeit des Polymeren gefunden. Diese Erkenntnis ermöglicht es, die Eignung der Gruppen A vor deren Einbau in das Polymer zu prüfen.

Gegenstand der Erfindung ist ein Verfahren zur Auswahl von optisch isotropen Polymeren für die reversible, optische Datenspeicherung, mit einer als Rückgrad wirkenden Hauptkette und davon abzweigenden kovalent gebundenen Seitengruppen der Formeln

$$-S^1-T^1-Q^1-A \qquad (I)$$

und

$$-S^2-T^2-Q^2-M \qquad (II)$$

worin

$S^1$, $S^2$ die Atome O, S oder den Rest $NR^0$ bedeuten,

$R^0$ Wasserstoff oder $C_1$-$C_4$-Alkyl bedeutet,

$T^1$, $T^2$ den Rest $(CH_2)_n$, der gegebenenfalls durch -O-, $-NR^0-$ oder $-OSiR^0{}_2O-$ unterbrochen sein kann und/oder gegebenenfalls durch Methyl oder Ethyl substituiert sein kann, bedeuten

$Q^1$, $Q^2$ eine direkte Bindung, -O-, -COO-, -OCO-, $-CONR^0-$, $-NR^0CO-$ oder $-NR^0-$ bedeuten oder

$S^1T^1Q^1$ oder $S^2T^2Q^2$ eine bivalente Gruppe der Formel

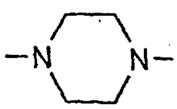

bedeuten

A eine Einheit, die elektromagnetische Strahlung aufnehmen kann, bedeutet

M eine mesogene, formanisotrope Einheit bedeutet und

n eine ganze Zahl von 2 bis 12 bedeutet,

dadurch gekennzeichnet, daß A einen Extinktionsmodul ΔΔE von größer als 0,2 (gemessen wie unten angegeben) besitzt.

Für die Zwecke der Erfindung wird der Wert ΔΔE an einer Verbindung der Formel $H\text{-}Q^1\text{-}A$ oder $HS^1T^1Q^1A$ gemessen, wobei $Q^1$, $S^1$, $T^1$ und A die oben angegebene Bedeutung besitzen und H für den Wasserstoffrest steht

Der Extinktionsmodul ΔΔE wird aus den Extinktionsänderungen ΔE der langwelligen Flanke der Absorptionsbande von 3 Lösungen folgender Stoffe bestimmt, nämlich

Lösung A $H\text{-}Q^1\text{-}A$ oder $HS^1T^1Q^1A$ wird in möglichst niedriger Konzentration in einem möglichst wenig polaren Lösungsmittel gelöst Hierbei wird die Konzentration vorzugsweise so gewählt, daß sich bei der Messung der Absorption eine möglichst steile Kante ergibt. Bei Farbstoffen, die in der Regel eine hohe molare Extinktion haben, ist diese Konzentration vorzugsweise $10^{-3}$ molar.

Lösung B In dem gleichen Lösungsmittel wird der Standard in möglichst hoher Konzentration, vzw. 1 molar, gelöst und die Absorptionskante aufgenommen.

Lösung C Diese Lösung enthält $H\text{-}Q^1\text{-}A$ oder $HS^1T^1Q^1A$ und den Standard in den Konzentrationen der Lösungen A und B.

Man erhält also 3 Absorptionskanten: Die des Standards, die i.a. kurzwellig liegt, sowie die Absorptionskante der Lösung A und die zu ihr langweilig parallel verschobene Kante der Lösung C.

An der längstwelligen Kante, also der Lösung C, definiert die zur Extinktion 0,8 gehörende Wellenlänge die Bezugswellenlänge λ. Man liest nun bei den Wellenlängen λ und λ + 50 jeweils die Extinktionswerte E der drei Lösungen A, B und C ab, wobei

$$E_{\text{Lösung C}} > E_{\text{Lösung A}} > E_{\text{Lösung B}}.$$

Zur Ermittlung des Wertes von ΔE bildet man für die Wellenlängen λ und λ+50 die folgenden Differenzen:

$$\Delta E_A = E\,(\lambda)_{\text{Lösung A}} - E\,(\lambda+50)_{\text{Lösung A}}$$

$$\Delta E_B = E\,(\lambda)_{\text{Lösung B}} - E\,(\lambda+50)_{\text{Lösung B}}$$

$$\Delta E_C = E\,(\lambda)_{\text{Lösung C}} - E\,(\lambda+50)_{\text{Lösung C}}$$

Aus diesen drei Differenzen erhält man die Extinktionszunahme des Farbstoffs durch die Anwesenheit des Standards als die Differenz ΔΔE:

$$\Delta\Delta E = \Delta E_C - (\Delta E_B + \Delta E_A)$$

Der Standard soll möglichst polar und/oder polarisierbar sein. Die Polarität des Lösungsmittels soll möglichst gering sein.

In einer bevorzugten Form wird als Standard 1,3-Dinitrobenzol verwendet und als Lösungsmittel Dioxan.

Wie oben erklärt, soll A elektromagnetische Strahlung absorbieren können. Die Absorptionsmaxima ($\lambda_{max}$) bevorzugter Gruppen A können im nahen IR, im Bereich des sichtbaren Lichts oder im UV, vorzugsweise im Wellenlängenbereich von 320-1500 nm, insbesondere von 350 bis 800 nm, liegen. Sofern im Rahmen dieser Erfindung die Begriffe "Chromophor" oder "Farbstoff" gebraucht werden, sind sie nicht auf den Wellenlängenbereich des sichtbaren Lichts beschränkt, sondern beziehen sich auf die Gruppen A.

Gruppen A, die ΔΔE-Werte über 0,2 ergeben, können aus den Resten folgender Farbstoffklassen ausgewählt werden (vgl. z.B. G. Ebner und D. Schulz, Textilfärberei und Farbstoffe, Springer-Verlag, Berlin Heidelberg 1989):

**I. Azofarbstoffe**

1. Monoazofarbstoffe, wie z.B.

    C.I. Mordant Yellow 1
    C.I. Mordant Blue 78
    C.I. Disperse Blue 79
    C.I. Disperse Yellow 5

2. Disazofarbstoffe, wie z.B.

    C.I. Mordant Yellow 16
    C.I. Disperse Yellow 23
    C.I. Basic Brown 1
    C.I. Disperse Yellow 7

**II. Chinoide Farbstoffe**

1. Chinoide Dispersions- und Beizenfarbstoffe, wie z.B.

    C.I. Disperse Orange 11
    C.I. Disperse Blue 5
    C.I. Disperse Blue 7
    C.I. Mordant Violet 26
    C.I. Mordant Blue 23

**III. Metallkomplex-Farbstoffe**

C.I. Ingrain Blue 14

**IV. Merochinoide Farbstoffe**

1. Diphenylmethanfarbstoffe, wie z.B.

    Basic Yellow 3

2. Triphenylmethanfarbstoffe, wie z.B.

    C.I. Basic Violet 3,
    C.I. Basic Green 4,
    C.I. Mordant Blue 1
    C.I. Mordant Blue 28

3. Chinoniminfarbstoffe, wie z.B.

    C.I. Solvent Blue 22

4. Acridinfarbstoffe, wie z.B.

    Acridinorange 2 G

5. Thioxanthenfarbstoffe, wie z.B.

Pyronin G, C.I. 45005

6. Phenazinfarbstoffe, wie z.B.

C.I. Solvent Blue 7

7. Phenoxazinfarbstoffe, wie z.B.

C.I. Mordant Blue 10

8. Phenothiazinfarbstoffe, wie z.B.

C.I. Mordant Blue 51

9. Quadratsäurefarbstoffe, wie z.B.

aus EP-A 0 145 401 bekannt

**V. Polymethinfarbstoffe**

umfassend Kationo-, Aniono- und Mero-(=Neutro)cyanine sowie Hemicyanine, wie z.B.

C.I. Disperse Yellow 31
C.I. Disperse Blue 354
C.I. Disperse Red 196
C.I. Disperse Yellow 99

**VI. Nitro- und Nitrosofarbstoffe** wie z.B.

C.I. Disperse Yellow 42
C.I. Disperse Yellow 1

**VII. Heterocyclische Farbstoffe**

1. Perinone, wie z.B.

C.I. Disperse Yellow 58

2. Naphthalimide, wie z.B.

Disperse Yellow 11

3. Chinophthalon, wie z.B.

Disperse Yellow 54, Disperse Yellow 64

4. Cumarine, wie z.B.

bekannt aus DE 15 94 845, 16 70 999, 20 65 076

5. Pyrazoline, wie z.B.

bekannt aus DE 11 55 418, 14 45 705, 14 19 329

6. Stilbenfarbstoffe, wie z.B.

C.I. Fluoreszent Brightener 30
C.I. Fluoreszent Brightener 46

Insbesondere können die Gruppen A aus den einbindigen Resten folgender Verbindungen ausgewählt werden:

$$Het^1 (= Z)_n = Het^2 \quad (I)$$

worin

$Het^1$

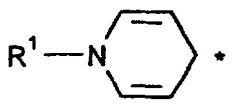

(1)

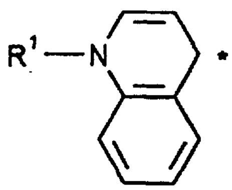

(2)

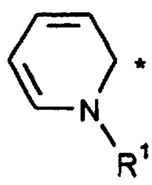

(3)

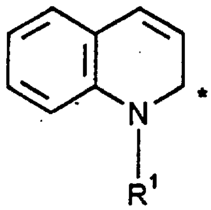

(4)

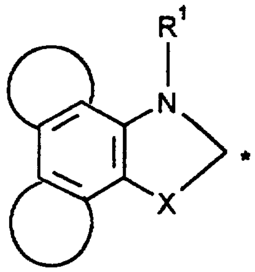

(5)

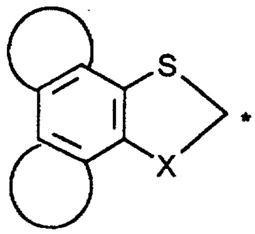

(6)

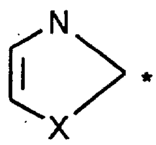

(7)

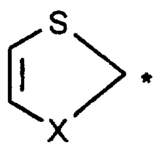

(8)

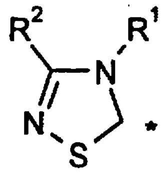

(9)

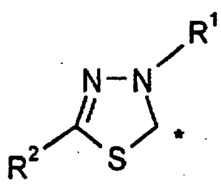

(10)

Z CH-CH oder N-N,

n Null oder 1,

Het2

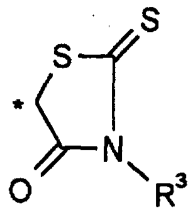

(11)

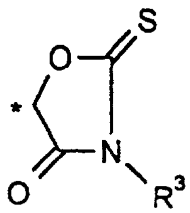

(12)

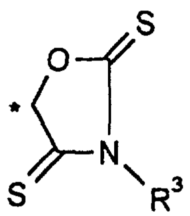

(13)

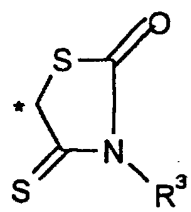

(14)

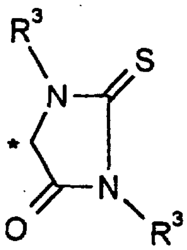

(15)

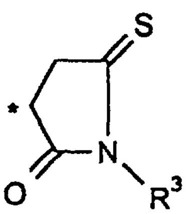

(16)

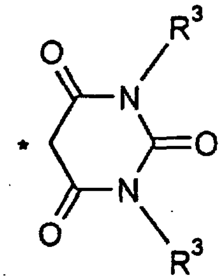

(17)

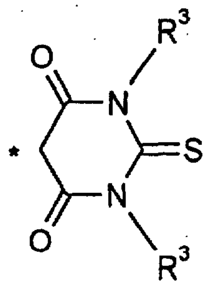

(18)

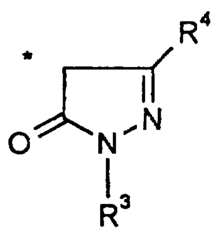

(19)

$R^1$ $C_1$-$C_6$-Alkyl, $C_2$-$C_6$-Alkenyl, $C_5$-$C_{10}$-Cycloalkyl, $C_7$-$C_{15}$-Aralkyl,
$R^2$ $C_1$-$C_6$-Alkyl, $C_1$-$C_4$-Alkoxy, $C_6$-$C_{12}$-Aryl, $C_6$-$C_{12}$-Aryloxy, $C_1$-$C_6$- Alkylthio, $C_6$-$C_{12}$-Arylthio, Mono- oder Di-$C_1$-$C_4$-alkylamino, $C_6$-$C_{12}$- Arylamino, $C_1$-$C_4$-Alkyl-$C_6$-$C_{12}$-arylamino, Chlor,
$R^3$ $C_1$-$C_6$-Alkyl, $C_2$-$C_6$-Alkenyl, $C_5$-$C_{10}$-Cycloalkyl, $C_6$-$C_{12}$-Aryl, $C_7$-$C_{15}$- Aralkyl,
$R^4$ $C_1$-$C_6$-Alkyl, $C_6$-$C_{12}$-Aryl, CN, $COOR^3$, CO-$R^3$,
X O, S, Se, $NR^1$, $CR^8_2$,
$R^8$ $C_1$-$C_6$-Alkyl bedeuten,

die Sternchen die Position der exocyclischen C=C-Doppelbindung charakterisieren und die bogenförmigen Linien in den Strukturen (5) und (6) Wasserstoff oder -CH=CH-CH=CH- bedeuten;

$$Het^1 (= Z)_n = Het^3 \qquad (II)$$

worin

$Het^3$

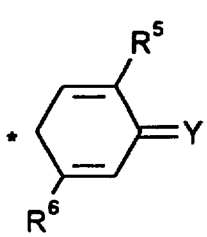

(20)

$R^5$ Wasserstoff, $C_1$-$C_6$-Alkyl, $C_1$-$C_6$-Alkoxy, Fluor, Chlor,

$R^6$ Wasserstoff, $C_1$-$C_6$-Alkyl, $C_1$-$C_6$-Alkoxy, Fluor, Chlor, CN, $NO_2$, $NHCOR^3$ oder $NHSO_2R^3$,

Y Sauerstoff, $C(CN)_2$, $C(CN)COOR^3$ oder

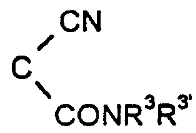 ,

bedeuten,

$Het^1$, Z, n, das Sternchen und $R^3$ die oben unter (I) angegebene Bedeutung besitzen und

$R^{3'}$ unabhängig von $R^3$ für die oben unter $R^3$ angegebene Bedeutung steht;

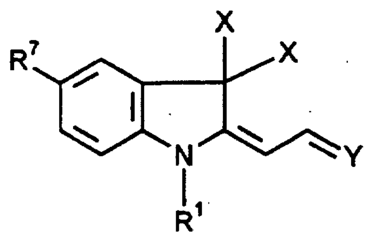

(III)

worin

Y die oben unter (II) angegebene Bedeutung - mit Ausnahme von Sauerstoff - und zusätzlich

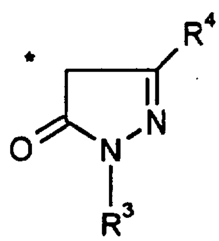

(21)

X, $R^1$, $R^3$, $R^4$ und das Sternchen die oben unter (I) angegebene Bedeutung besitzen und
$R^7$ Wasserstoff, $C_1$-$C_6$-Alkyl, $C_1$-$C_6$-Alkoxy, $COOR^3$, Chlor, $NO_2$, CN bedeuten;

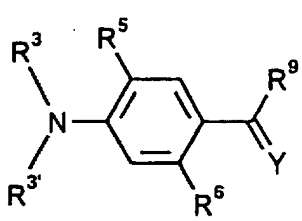

(IV)

worin

$R^3$, $R^{3'}$ unabhängig voneinander die oben unter (I) für $R^3$ angegebene Bedeutung,

$R^5$, $R^6$ die oben unter (II) angegebene Bedeutung und

Y die oben unter (III) angegebene Bedeutung besitzen,

$R^9$ Wasserstoff, $C_1$-$C_6$-Alkyl, $C_6$-$C_{12}$-Aryl, CN oder $COOR^3$ und <u>außerdem</u> zusätzlich

$R^{3'}$ Wasserstoff,

$NR^3R^{3'}$,

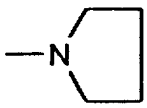 , 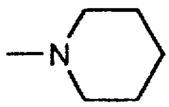 , 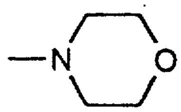 , 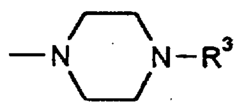

(22) (23) (24) (25)

und

$R^{3'}$ und $R^5$ zusammen $-(CH_2)_2-$ , $-(CH_2)_3-$ , $-C(CH_3)_2-CH_2-CH(CH_3)-$ oder $-OCH_2CH_2-$ bedeuten;

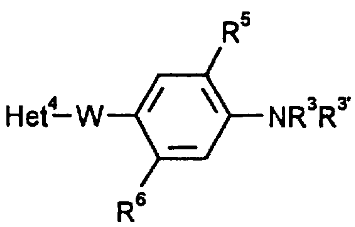 (V)

worin

$Het^4$

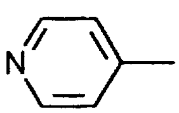 , 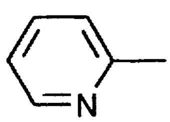 , 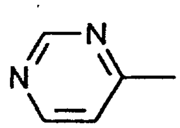 ,

(26) (27) (28)

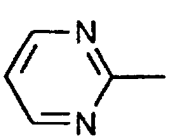 , 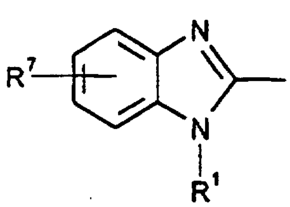 , 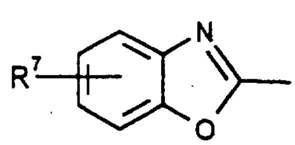 ,

(29) (30) (31)

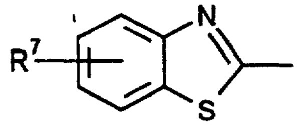 , 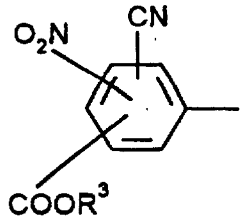 ,

(32) (33)

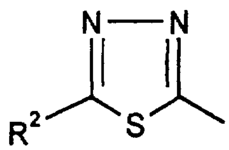 , 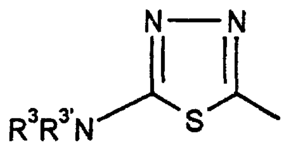 , 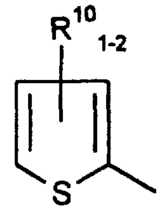 ,

(34) (35) (36)

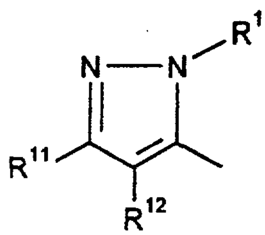 , 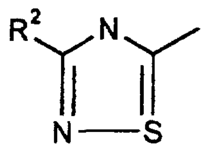 , 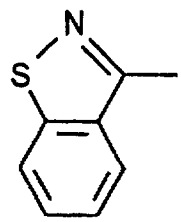 ,

(37) (38) (39)

W -N=N- oder

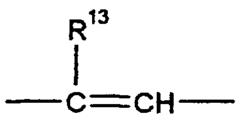 ,

$R^{10}$ CN, $NO_2$ oder $COOR^3$,

$R^{11}$ $C_1$-$C_6$-Alkyl, $C_1$-$C_6$-Alkoxy, Chlor, Amino, $C_1$-$C_7$-Acylamino, Di-$C_1$-$C_4$-alkylamino,

$R^{12}$ $C_1$-$C_6$-Alkyl, $C_5$-$C_{12}$-Aryl, CN, $COOR^3$,

$R^{13}$ Wasserstoff, CN oder $NO_2$ bedeuten und $R^1$, $R^2$, $R^7$, $R^3$, $R^{3'}$, $R^5$, $R^6$ die bei (I), (III) und (IV) angegebene Bedeutung besitzen;

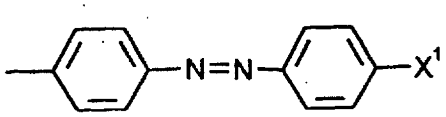 (VI),

worin

$X^1$ Wasserstoff, Hydroxyl, Mercapto, $CF_3$, $CCl_3$, $CBr_3$, Halogen, Cyan, Nitro, $COOR^{19}$, $C_1$-$C_6$-Alkyl, $C_5$-$C_{12}$-Cycloalkyl, $C_1$-$C_{12}$-Alkoxy, $C_1$-$C_{12}$-Alkylthio, $C_6$-$C_{12}$-Aryl, $C_6$-$C_{12}$-Aryloxy, $C_6$-$C_{12}$-Arylthio, $C_1$-$C_6$-Alkylsulfonyl, $C_6$-$C_{12}$-Arylsulfonyl, Aminosulfonyl, $C_1$-$C_6$-Alkylaminosulfonyl, Phenylaminosulfonyl, Aminocarbonyl, $C_1$-$C_6$-Alkylaminocarbonyl, Phenylaminocarbonyl, $NR^{19}$,$R^{20}$, NH-CO-$R^{19}$, NH-$SO_2$-$R^{19}$, NH-CO-$NR^{19}R^{20}$, NH-CO-O-$R^{19}$ oder $SO_2$-$CF_3$ bedeutet, worin $R^{19}$ und $R^{20}$ unabhängig voneinander für Wasserstoff, $C_1$-$C_4$-Alkyl oder Phenyl stehen, wobei einer der Reste $R^1$ bis $R^{13}$ (einschließlich $R^{3'}$), insbesondere einer der Reste $R^1$ und $R^3$, eine Einfachbindung, die die Anknüpfung an die Gruppe $Q^1$ ermöglicht, darstellt, wobei im Falle der Reste $R^1$, $R^3$ und $R^{3'}$, $Q^1$ dann für eine Einfachbindung steht.

Besonders bevorzugte Verbindungen (V) entsprechen den Formeln

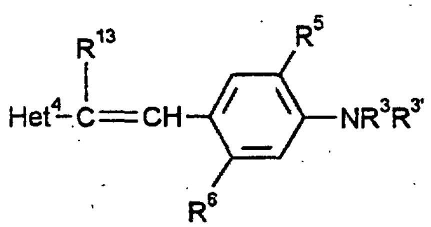

(Va)

worin $Het^4$ für eine der Strukturen 26 bis 33 steht und

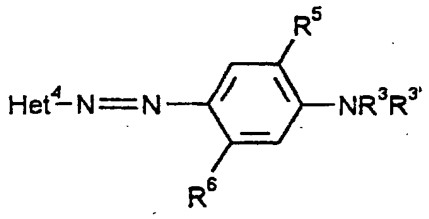

(Vb)

worin $Het^4$ für eine der Strukturen 26, 27, 30, 32 oder 34 bis 39 steht.

Wie eingangs geschildert, ist die Gruppe über die Zwischenglieder $Q^1$, $T^1$ und $S^1$ an die Hauptketten der ausgewählten Polymeren gebunden. Die oben genannten Farbstoffe können diese Zwischenglieder bereits ganz oder teilweise enthalten; vgl. Legende der Substituenten. Unter als A geeigneten Farbstoffresten sind also die jeweils um solche bereits enthaltenen Zwischenglieder verkürzten Farbstoffe zu verstehen. Andererseits können Farbstoffe, soweit sie die Zwischenglieder $Q^1$, $T^1$, $S^1$ nicht oder nur teilweise enthalten, durch entsprechende Reaktion zu den gewünschten reaktiven Derivaten umgesetzt werden, die dann zum Aufbau der ausgewählten Polymeren geeignet sind.

Die Einheit -$Q^1$-$T^1$-$S^1$- kann also z.B. für -$NR^0$-$(CH_2)_n$-O- stehen. So lassen sich bei gegebener Gruppe A geeignete Verbindungen der Struktur A-$NR^0$-$(CH_2)_n$-OH zur Einführung von A in die ausgewählten Polymeren oder in die zu polymerisierenden Monomeren bereitstellen. (Bei anderer Bedeutung von $Q^1$, $T^1$, $S^1$ gilt Analoges). Geeignete solche Verbindungen können also beispielsweise die folgenden Verbindungen umfassen:

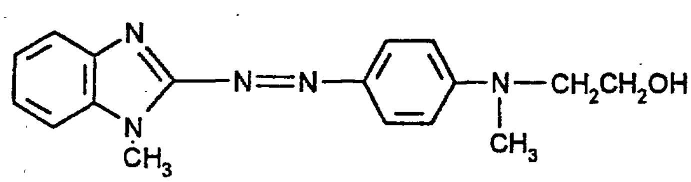

(VII)

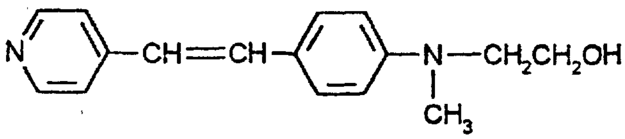

(VIII)

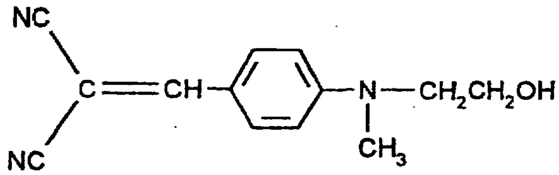

(IX)

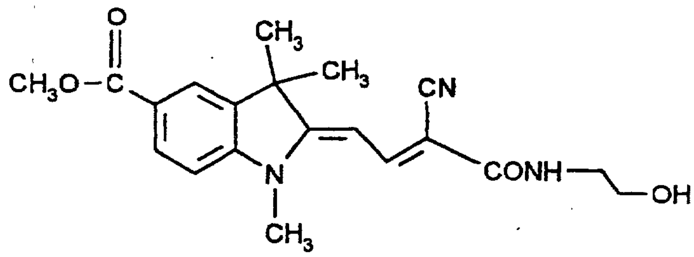

(X)

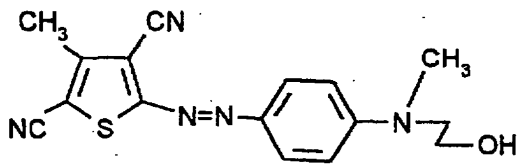

(XI)

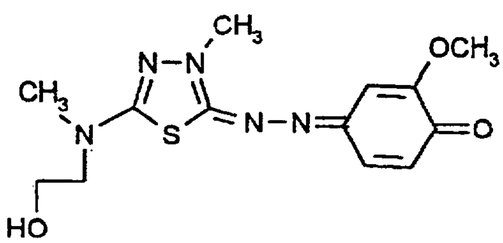

(XII)

In Ergänzung der obigen Definitionen bedeutet pro Rest A einer der Substituenten $R^1$ bis $R^{13}$ (einschließlich $R^{3'}$), insbesondere einer der Reste $R^1$ und $R^3$, eine Einfachbindung, die die Anknüpfung an die Gruppe $Q^1$ ermöglicht.

Die Verbindungen

(1) $A - Q^1 - T^1 - S^1 - H$
(2) $A - Q^1 - T^1 - S^1 - OC - CH = CH_2$
(3) $A - Q^1 - T^1 - S^1 - OC - C(CH_3) = CH_2$
(4) $A - Q^1 - T^1 - S^1 - (CH_2)_n - OH$
(5) $A - Q^1 - T^1 - S^1 - CH_2 - CHOH\text{-}CH_3$
(6) $A - Q^1 - T^1 - S^1 - CH_2\text{-}C(CH_3)_2\text{-}CH_2\text{-}OH$

worin A, $Q^1$, $T^1$, $S^1$ die oben angegebenen Bedeutungen besitzen und n für eine ganze Zahl von 2 bis 12 steht, und

die Acrylate und Methacrylate der Verbindungen 4 bis 6 können analog den Verfahren für ähnliche Verbindungen hergestellt werden.

Die erfindungsgemäß ausgewählten Polymere enthalten keine Gruppen A der Formel

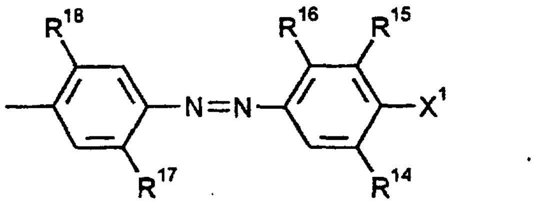

(XII)

worin

$R^{14}$ bis $R^{16}$ unabhängig voneinander $C_1$-$C_6$-Alkyl, Hydroxyl, $C_1$-$C_6$-Alkoxy, Phenoxy, $C_1$-$C_6$-Alkylthio, Phenylthio, Halogen, $CF_3$, $CCl_3$, $CBr_3$, Nitro, Cyan, $C_1$-$C_6$-Alkylsulfonyl, Phenylsulfonyl, $COOR^1$, Aminosulfonyl, $C_1$-$C_6$-Alkylaminosulfonyl, Phenylaminosulfonyl, Aminocarbonyl, $C_1$-$C_6$-Alkylaminocarbonyl, Phenylaminocarbonyl bedeuten,

$R^{17}$ Halogen, $C_1$-$C_6$-Alkyl, Hydroxyl, $C_1$-$C_6$-Alkoxy, Phenoxy, $C_1$-$C_4$-Acylamino, $C_1$-$C_4$-Alkylsulfonylamino bedeutet,

$R^{18}$ Halogen, $C_1$-$C_6$-Alkyl, Hydroxyl, $C_1$-$C_6$-Alkoxy, Phenoxy bedeutet,

$X^1$ Wasserstoff, Hydroxyl, Mercapto, $CF_3$, $CCl_3$, $CBr_3$, Halogen, Cyan, Nitro, $COOR^{19}$, $C_1$-$C_6$-Alkyl, $C_5$-$C_{12}$-Cycloalkyl, $C_1$-$C_{12}$-Alkoxy, $C_1$-$C_{12}$-Alkylthio, $C_6$-$C_{12}$-Aryl, $C_6$-$C_{12}$-Aryloxy, $C_6$-$C_{12}$-Arylthio, $C_1$-$C_6$-Alkylsulfonyl, $C_6$-$C_{12}$-Arylsulfonyl, Aminosulfonyl, $C_1$-$C_6$-Alkylaminosulfonyl, Phenylaminosulfonyl, Aminocarbonyl, $C_1$-$C_6$-Alkylaminocarbonyl, Phenylaminocarbonyl, $NR^{19}$,$R^{20}$, NH-CO-$R^{19}$, NH-$SO_2$-$R^{19}$, NH-CO-$NR^{19}R^{20}$, NH-CO-O-$R^{19}$ oder $SO_2$-$CF_3$ bedeutet, worin $R^{19}$ und $R^{20}$ unabhängig voneinander für Wasserstoff, $C_1$-$C_4$-Alkyl oder Phenyl stehen.

Bevorzugte mesogene Einheiten M entsprechen der Formel

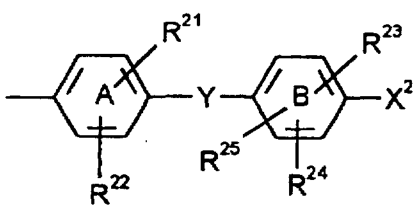

(XIV)

worin

$R^{21}$ bis $R^{25}$ unabhängig voneinander Wasserstoff, Halogen, $C_1$-$C_4$-Alkyl, $C_1$-$C_4$-Alkoxy, $CF_3$, Nitro, $SO_2CH_3$, $SO_2NH_2$ oder Cyan bedeuten, wobei mindestens einer der Substituenten $R^{21}$ bis $R^{25}$ ungleich Wasserstoff sein muß,

Y eine direkte Bindung, -COO-, -OCO-, -CONH-, -NHCO-, -O-, -NH-, -N($CH_3$)- oder -N=N- und

$X^2$ Wasserstoff, Hydroxyl, Mercapto, $CF_3$, $CCl_3$, $CBr_3$, Halogen, Cyan, Nitro, $COOR^{19}$, $C_1$-$C_6$-Alkyl, $C_5$-$C_{12}$-Cycloalkyl, $C_1$-$C_{12}$-Alkoxy, $C_1$-$C_{12}$-Alkylthio, $C_6$-$C_{12}$-Aryl, $C_6$-$C_{12}$-Aryloxy, $C_6$-$C_{12}$-Arylthio, $C_1$-$C_6$-Alkylsulfonyl, $C_6$-$C_{12}$-Arylsulfonyl, Aminosulfonyl, $C_1$-$C_6$- Alkylaminosulfonyl, Phenylaminosulfonyl, Aminocarbonyl, $C_1$-$C_6$- Alkylaminocarbonyl, Phenylaminocarbonyl, $NR^{19}R^{20}$, NH-CO-$R^{19}$, NH-$SO_2$-$R^{19}$, NH-CO-$NR^{19}R^{20}$, NH-CO-O-$R^{12}$ oder $SO_2$-$CF_3$
bedeutet, worin $R^{19}$ und $R^{20}$ unabhängig voneinander für Wasserstoff, $C_1$-$C_4$-Alkyl oder Phenyl stehen, wobei aber

$R^{21}$ bis $R^{25}$ alle für Wasserstoff stehen wenn A einem Rest der Formel (VI) entspricht.

Die bevorzugten ausgewählten Polymere enthalten allein wiederkehrende Einheiten mit den Seitengruppen I und II, und zwar vorzugsweise solche der Formeln

(XV) 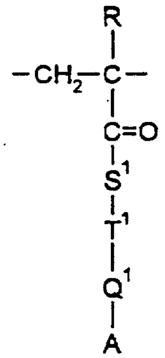 und 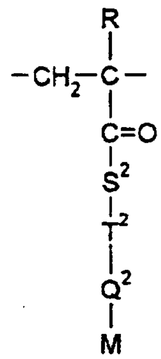 (XVI)

mit R = H oder Methyl.

Bevorzugte Beispiele der Gruppen $T^1$ und $T^2$ sind unabhängig voneinander

$$-(CH_2)_n- \;,\; -(CH_2)_m-CH(CH_3)-(CH_2)_o- \;,\; -(CH_2)_m-C(CH_3)_2-(CH_2)_o- \;,$$

$$-(CH_2)_m-O-(CH_2)_o- \text{ und } -(CH_2)_m-N(CH_3)-(CH_2)_o- \;,$$

worin

n eine ganze Zahl von 2 bis 6 ist und
m und o unabhängig voneinander ganze Zahlen von 0 bis 2 sind, deren Summe mindestens 1 beträgt.

Ebenfalls bevorzugt ist $S^1T^1Q^1$ oder $S^2T^2Q^2$ =

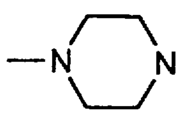

Als bevorzugte Seitengruppen II kommen insbesondere solche der Formel

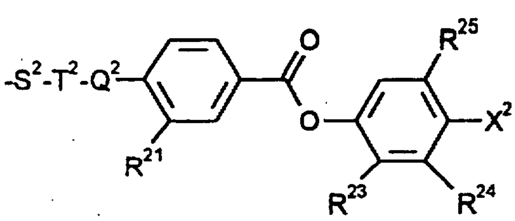 (XVII)

worin

$X^2$, $T^2$, $Q^2$ und $R^{21}$ bis $R^{25}$ die oben angegebenen Bedeutungen haben, in Frage.

Bevorzugte Monomere zur Einführung der Seitengruppen II entsprechen der Formel

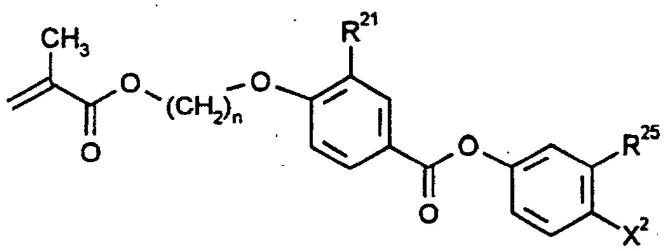

(XVIII)

worin

n eine ganze Zahl von 2 bis 6,
$R^{21}$, $R^{25}$ unabhängig voneinander für H, F, Cl, Br, OH, $OCH_3$, $CH_3$, $CF_3$, $NO_2$ oder CN stehen und

wenigstens einer der Substituenten $R^{21}$ und $R^{25}$ ungleich H sein muß und

$X^2$ H, F, Cl, Br, CN, $NO_2$, $COOR^{19}$, $C_5$-$C_{12}$-Cycloalkyl, $C_1$-$C_{12}$-Alkoxy oder $C_6$-$C_{12}$-Aryl bedeuten

und $R^{19}$, $R^{21}$ und $R^{25}$ die oben angegebene Bedeutung haben.

Beispiele für derartige Monomere sind

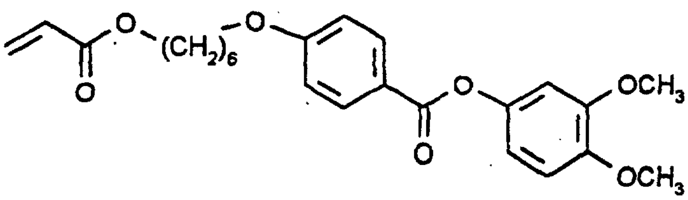

(XIX)

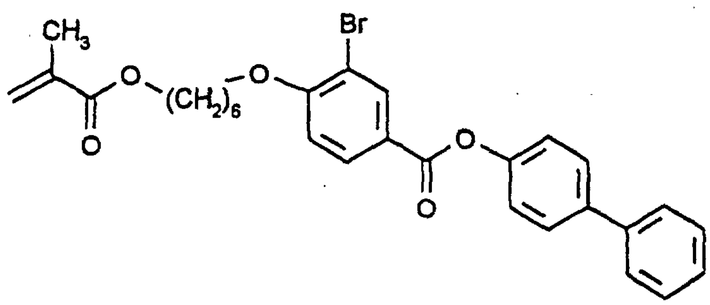

(XX)

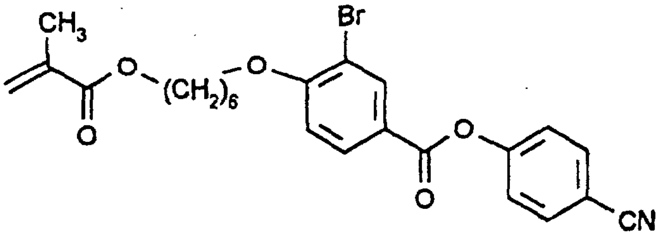

(XXI)

Vorzugsweise wird die Hauptkette der Seitengruppenpolymeren von Monomeren, die die Seitengruppen (I) tragen, von Monomeren, die die Seitengruppe (II) tragen und gegebenenfalls weiteren Monomeren gebildet, wobei insbesondere der Anteil der Monomeren, die die Seitengruppe (I) aufweisen, 25 bis 80 Mol-%, vorzugsweise 30 bis 70 Mol-%, der Anteil der Monomeren, die die Seitengruppe (II) aufweisen, 20 bis 75 Mol-%, vorzugsweise 30 bis 70 Mol-%, und der Anteil der weiteren Monomeren 0 bis 50 Mol-% betragen, jeweils bezogen auf die Summe sämtlicher eingebauter Monomereinheiten.

Als "weitere" wiederkehrende Einheiten kommen alle Bausteine in Betracht, die sich chemisch in das Seitengruppenpolymer einbauen lassen. Sie dienen im wesentlichen lediglich dazu, die Konzentration der Seitengruppen I und II im Polymer zu verringern und bewirken also quasi einen "Verdünnungs" effekt. Im Falle von Poly(meth)acrylaten umfassen die "weiteren" Monomeren ethylenisch ungesättigte copolymerisierbare Monomere, die bevorzugt $\alpha$-substituierte Vinylgruppen oder $\beta$-substituierte Allylgruppen tragen, bevorzugt Styrol; aber auch beispielsweise kernchlorierte und -alkylierte bzw. -alkenylierte Styrole, wobei die Alkylgruppen 1 bis 4 Kohlenstoffatome enthalten können, wie z.B. Vinyltoluol, Divinylbenzol, $\alpha$-Methylstyrol, tert.-Butylstyrole, Chlorstyrole; Vinylester von Carbonsäuren mit 2 bis 6 Kohlenstoffatomen, bevorzugt Vinylacetat; Vinylpyridin, Vinylnaphthalin, Vinylcyclohexan, Acrylsäure und Methacrylsäure und/oder ihre Ester (vorzugsweise Vinyl-, Allyl- und Methallylester) mit 1 bis 4 Kohlenstoffatomen in der Alkoholkomponente, ihre Amide und Nitrile, Maleinsäureanhydrid, -halb- und -diester mit 1 bis 4 Kohlenstoffatomen in der Alkoholkomponente, -halb- und -diamide und cyclische Imide wie N-Methylmaleinimid oder N-Cyclohexylmaleinimid; Allylverbindungen wie Allylbenzol und Allylester, wie Allylacetat, Phthalsäurediallylester, Isophthalsäurediallylester, Fumarsäurediallylester, Allylcarbonate, Diallylcarbonate, Triallylphosphat und Triallylcyanurat

Bevorzugte "weitere" Monomere entsprechen der Formel

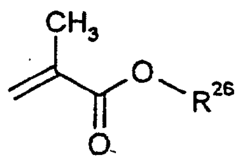

(XXII)

worin

$R^{26}$ für einen gegebenenfalls verzweigten $C_1$-$C_6$-Alkylrest oder einen wenigstens einen weiteren Acrylrest enthaltenden Rest steht.

Die ausgewählten Polymere können auch mehr als eine Seitengruppe, die unter die Definition von (I) fällt, oder mehr als eine Seitengruppe, die unter die Definition von (II) fällt, oder mehrere Seitengruppen sowohl der Definition von (I) als auch von (II) enthalten, davon kann auch eine Seitengruppe der Definition (I) der Formel XIII entsprechen. Im Falle des Vorhandenseins von Seitenketten der Struktur (I) und (II) hat in vorteilhafter Weise wenigstens eine Gruppe $Q^1$ bzw. $Q^2$ die Bedeutung -O-$C_6H_4$-COO- oder -O-$C_6H_4$-CONR$^{19}$.

Die ausgewählten Polymere besitzen vorzugsweise Glasübergangstemperaturen Tg von mindestens 40°C. Die Glasübergangstemperatur kann beispielsweise nach B. Vollmer, Grundriß der Makromolekularen Chemie, S. 406-410, Springer-Verlag, Heidelberg 1962, bestimmt werden.

Die ausgewählten Polymere besitzen im allgemeinen ein als Gewichtsmittel bestimmtes Molekulargewicht von 5.000 bis 2.000.000, vorzugsweise von 8.000 bis 1.500.000, bestimmt durch Gelpermeationschromatographie (geeicht mit Polystyrol).

Die Strukturelemente mit hoher Formanisotropie und hoher Anisotropie der molekularen Polarisierbarkeit sind die Voraussetzung für hohe Werte der optischen Anisotropie. Durch die Struktur der Polymeren werden die zwischenmolekularen Wechselwirkungen der Strukturelemente (I) und (II) so eingestellt, daß die Ausbildung flüssigkristalliner Ordnungszustände unterdrückt wird und optisch isotrope, transparente nichtstreuende Filme hergestellt werden können. Andererseits sind die zwischenmolekularen Wechselwirkungen dennoch stark genug, daß bei Bestrahlung mit polarisiertem Licht ein photochemisch induzierter, kooperativer, gerichteter Umorientierungsprozeß der photochromen und der nichtphotochromen Seitengruppen bewirkt wird.

Bevorzugt treten zwischen den Seitengruppen (I) und (II) Wechselwirkungskräfte auf, die ausreichen, daß die photoinduzierte Konfigurationsänderung der Seitengruppe (I) eine gleichgerichtete - sogenannte kooperative - Umorientierung der Seitengruppe (II) bewirkt.

In den optisch isotropen amorphen photochromen Polymeren können extrem hohe Werte der optischen Anisotropie induziert werden ($\Delta n$ = 0,01 bis 0,2, vorzugsweise 0,01 bis 0,1). Die Werte sind denen vergleichbar, die in Monodomänen flüssigkristalliner Polymerer erhalten wurden, oder sind sogar größer als diese. Sie sind signifikant größer im Vergleich zu amorphen Polymeren ohne diese Strukturelemente.

Durch den Einfluß von aktinischem Licht werden in den Seitengruppenpolymeren Ordnungszustände generiert und modifiziert und damit ihre optischen Eigenschaften moduliert.

Als Licht wird vorzugsweise linear polarisiertes Licht verwendet, dessen Wellenlänge im Bereich der Absorptionsbande der photoinduzierbar konfigurationsveränderlichen Seitengruppen (I) liegt.

Die Herstellung der Seitengruppenmonomeren und ihre Polymerisation können nach literaturbekannten Verfahren durchgeführt werden (beispielsweise DD 276 297, DE 3 808 430, Makromolekulare Chemie 187, 1327-1334 (1984), SU 887 574, Europ. Polym. 18, 561 (1982) und Liq. Cryst. 2, 195 (1987)).

Die Herstellung isotroper Filme gelingt, ohne daß aufwendige Orientierungsverfahren unter Nutzung externer Felder und/oder von Oberflächeneffekten notwendig sind. Sie lassen sich durch Spincoating, Tauchen, Gießen oder andere technologisch leicht beherrschbare Beschichtungsverfahren auf Unterlagen aufbringen, durch Pressen oder Einfließen zwischen zwei transparente Platten bringen oder einfach als selbsttragenden Film durch Gießen oder Extrudieren präparieren. Solche Filme lassen sich durch schlagartiges Abkühlen, d.h. durch eine Abkühlungsrate von > 100 K/min, oder durch rasches Abziehen des Lösungsmittels auch aus flüssig-kristallinen Polymeren herstellen, die Strukturelemente im beschriebenen Sinne enthalten.

Die Schichtdicke liegt vorzugsweise zwischen 0,1 µm und 1 mm, besonders zwischen 0,5 und 100 µm.

Die lichtinduzierte Orientierung der Seitengruppen bzw. das Einschreiben von Information erfolgt durch Bestrahlung mit für die photoinduzierbar konfigurationsveränderliche Gruppe A geeignetem aktinischem Licht. Dies führt zu einer winkelabhängigen Photoselektion, die eine Umorientierung der photochromen Gruppen und - durch einen kooperativen Effekt - eine kontinuierliche, gleichgerichtete Umorientierung der permanent formanisotropen Seitengruppen M bis maximal senkrecht zum elektrischen Vektor des Anregungslichtes bewirkt.

Die Lichtexposition kann flächig oder lokal mit linear polarisiertem, kohärenten oder nicht kohärentem, monochromatischem Licht erfolgen, dessen Wellenlänge im Absorptionsbereich der photoinduzierbar konfigurationsveränderlichen Seitengruppen A liegt.

Die Information kann mit einem Laser punktförmig oder mit einem Laser oder einer Lampe flächig unstrukturiert oder unter Verwendung einer Maske oder durch die Einschreibung eines holographischen Brechungsindexgitters bei einer Intensität von 1 bis 500 $mW/cm^2$ in einer Zeit zwischen 1 und 30 000 sec. eingeschrieben werden.

Der Umorientierungsprozeß ist außerordentlich wirksam. Die unterhalb Tg erzielbare Doppelbrechungsänderung $\Delta n$ beträgt vorzugsweise 0,01 bis 0,20, insbesondere 0,05 bis 0,10.

Die hohen Werte der photochemisch induzierten Doppelbrechung und des photochemisch induzierten Dichroismus resultieren aus der molekularen Struktur der Seitengruppen, dem kooperativen Mechanismus der lichtinduzierten Orientierung zu einem Zustand gleicher makroskopischer Orientierung der photochromen und nichtphotochromen, aber permanent formanisotropen Seitengruppen.

Die Vorzugsorientierung ist frei wählbar; sie hängt allein von der Wahl der Richtung des elektrischen Vektors des Anregungslichtes in Bezug auf den Polymerkörper ab. Das Ausmaß der Orientierung ist bei konstanter Temperatur und Wellenlänge allein von der eingestrahlten Energie abhängig, die entweder über die Zeit oder in gewissen Grenzen die Leistung der Lichtquelle variiert werden kann. Es sind somit die Orientierung, die Doppelbrechung und der Dichroismus frei wählbare Parameter, die sich unter gleichbleibenden Randbedingungen bei wiederholtem Einschreiben und Löschen exakt reproduzieren lassen.

In den Seitenkettenpolymeren kann eine reproduzierbare, definierte, kontinuierlich durchstimmbare, langzeitstabile Doppelbrechung erzeugt werden. Sie läßt sich in Transmission im polarisierten Licht als definierter Kontrast darstellen. Bei Verwendung von Polymeren, deren Seitengruppen dichroitische Eigenschaften haben, läßt sich entsprechend ein Dichroismus der Absorption oder der Emission reproduzierbar, definiert und kontinuierlich durchstimmbar erzeugen. Durch einheitliche Bestrahlungsbedingungen wird im gesamten Polymerfilm eine einheitliche Orientierung erzeugt. Bei lokaler Variation der Bestrahlungsbedingungen wie Energiedosis und Polarisationsrichtung wird ein hinsichtlich der Vorzugsorientierung der Seitengruppen strukturierter Film erzeugt, was zu Pixeln mit unterschiedlicher optischer Anisotropie führt.

Die Vorzugsrichtung in der Orientierungsverteilung des optisch anisotropen Films kann durch Belichten mit unpolarisiertem aktinischem Licht wieder rückgängig gemacht und die optische Isotropie entlang der Flächennormalen wieder hergestellt werden. Erneute Bestrahlung mit der gleichen Quelle, jedoch veränderter Lage des elektrischen Vektors in Bezug auf den Polymerfilm führt zu einer Modifizierung der Richtung und Größe der optischen Anisotropie. Auf diese Weise kann zwischen verschiedenen Zuständen bezüglich der Richtung und Größe der optischen Anisotropie wiederholt geschaltet werden.

Auf der Grundlage dieser Effekte steht mit den beschriebenen Polymeren im Prinzip ein Medium für die reversible, optische Datenspeicherung zur Verfügung. Wie bei der Herstellung der Filme entfallen auch nach dem Löschen der Information alle Maßnahmen zur Wiederherstellung der Monodomäne.

Die Polymeren lassen sich zur digitalen oder analogen Datenspeicherung im weitesten Sinne, beispielsweise zur optischen Signalverarbeitung, zur Fourier-Transformation und -Faltung oder in der kohärenten optische Korrelationstechnik, verwenden. Die laterale Auflösung wird durch die Wellenlänge des Ausleselichts begrenzt. Sie erlaubt eine Pixelgröße von 1 bis 100 µm.

Diese Eigenschaft macht die Polymeren zur Verarbeitung von Bildern und zur Informationsverarbeitung mittels Hologrammen besonders geeignet, deren Reproduktion durch Ausleuchten mit einem Referenzstrahl erfolgen kann. Analog läßt sich das Interferenzmuster zweier monochromatischer kohärenter Lichtquellen mit konstanter Phasenbeziehung speichern und durch den Zusammenhang zwischen dem elektrischen Vektor des Lichts und der damit verbundenen Vorzugsrichtung im Speichermedium eine höhere Speicherdichte erzeugen. Entsprechend lassen sich dreidimensionale holographische Bilder speichern. Das Auslesen erfolgt durch Beleuchtung des Hologramms mit monochromatischem, kohärentem Licht. Bei der analogen Speicherung können Werte der Grauskala kontinuierlich und ortsaufgelöst eingestellt werden. Das Auslesen analog gespeicherter Information geschieht im polarisierten Licht, wobei man je nach Stellung der Polarisatoren das positive oder das negative Bild hervorholen kann. Hierbei kann einerseits der durch die Phasenverschiebung von ordentlichem und außerordentlichem Strahl erzeugte Kontrast des Films zwischen zwei Polarisatoren genutzt werden, wobei die Ebenen des Polarisators vorteilhaft einen Winkel von 45° zur Polarisationsebene des Einschreiblichts bilden und die Polarisationsebene des Analysators entweder senkrecht oder parallel zu der des Polarisators steht. Eine andere Möglichkeit besteht in der Detektion des durch induzierte Doppelbrechung verursachten Ablenkwinkels des Leselichts.

Die Polymeren lassen sich als optische Komponenten verwenden, die passiv oder optisch schaltbar sein können. So kann die hohe lichtinduzierte optische Anisotropie zur Modulierung der Intensität und/oder des Polarisationszustandes von Licht benutzt werden. Entsprechend kann man aus einem Polymerfilm durch holographische Strukturierung Komponenten herstellen, die Abbildungseigenschaften haben, die mit Linsen oder Gittern vergleichbar sind. Die Polymeren sind weiterhin zur Herstellung von Polarisatoren einsetzbar.

Weiterer Gegenstand der Erfindung ist also die Verwendung der erfindungsgemäß ausgewählten Polymeren für optische Bauelemente. Als solche optischen Bauelemente sind auch Polarisatoren anzusehen.

Die ausgewählten Polymere können in üblicher Weise durch radikalische Copolymerisation der Monomeren in geeigneten Lösungsmitteln, wie z.B. aromatischen Kohlenwasserstoffen wie Toluol oder Xylol, aromatischen Halogenkohlenwasserstoffen wie Chlorbenzol, Ethern wie Tetrahydrofuran und Dioxan, Ketonen wie Aceton und Cyclohexanon, und/oder Dimethylformamid in Gegenwart radikalliefernder Polymerisationsinitiatoren, wie z.B. Azodiisobutyronitril oder Benzoylperoxid, bei erhöhten Temperaturen, in der Regel bei 30 bis 130°C, vorzugsweise bei 40 bis 70°C, möglichst unter Wasser- und Luftausschluß hergestellt werden. Die Isolierung kann durch Ausfällen mit geeigneten Mitteln, z.B. Methanol erfolgen. Die Produkte können durch Umfällen, z.B. mit Chloroform/Methanol gereinigt werden.

Die ausgewählten Polymere können selbsttragende Filme bilden.

Vorzugsweise werden sie aber auf Trägermaterialien, beispielsweise Glas oder Kunststoffolien, aufgebracht. Dies kann durch verschiedene an sich bekannte Techniken geschehen, wobei das Verfahren danach ausgewählt wird, ob eine dicke oder dünne Schicht gewünscht wird. Dünne Schichten können z.B. durch Spincoaten oder Rakeln aus Lösungen oder der Schmelze, dickere durch Füllen von vorgefertigten Zellen, Schmelzpressen oder Extrudieren erzeugt werden.

Die Prozentangaben in den nachfolgenden Beispielen beziehen sich - falls nicht anders angegeben - jeweils auf das Gewicht.

## Beispiele

### Beispiel 1

In Dioxan werden von 4-(N-Methyl-N-hydroxyethyl)-amino-4'-nitro-azobenzol (MG 300) eine $10^{-3}$ molare Lösung (Lösung A) und von 1.3-Dinitrobenzol (MG 168) eine 1 molare Lösung (Lösung B) hergestellt. Das 1.3-Dinitrobenzol muß sehr sauber sein und ggf. durch Kristallisation aus Dioxan gereinigt werden. Zur Herstellung der Lösung C wiegt man 30 mg des Farbstoffs in einen 100 ml Pipettierkolben und füllt mit der Lösung B auf. Lösung C zeigt eine deutlich wahrnehmbare Farbvertiefung gegenüber den Lösungen A und B. Von den drei Lösungen werden die langwelligen Absorptionskanten mit einem UV-VIS-Spektrophotometer (Perkin-Elmer, Modell Lambda 3) in einer 1 cm-Küvette ausgehend von langen Wellenlängen bis zum Anschlag des Schreibers registriert. Man erhält 3 gut voneinander getrennte, im wesentlichen parallel verlaufende Absorptionskanten. Der Vollausschlag des Schreibers definiert die Extinktion 1,0. Die Kante der Lösung C erreicht den Extinktionswert $E_{\text{Lösung C}}$ = 0.8 bei $\lambda$ = 575 nm, woraus sich die zweite Ablesewellenlänge zu $\lambda$+50 = 625 nm ergibt. Die zugehörigen Extinktionswerte sind bei $\lambda$ = 575 nm für $E_{\text{Lösung A}}$ = 0,185 und für $E_{\text{Lösung B}}$ = 0,06. Bei 625 nm sind die Extinktionen $E_{\text{Lösung C}}$ = 0,070, $E_{\text{Lösung A}}$ = 0,05 und $E_{\text{Lösung B}}$ = 0,025. Hieraus ergeben sich die Extinktionsdifferenzen $\Delta E_C$ = 0,73, $\Delta E_A$ = 0,05 und $\Delta E_B$ = 0,025. Hieraus folgt für $\Delta\Delta E$ = 0,56. Nach der gleichen Methode findet man für die folgenden Verbindungen:

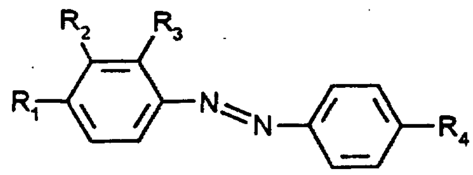

| Beispiel | $R^1$ | $R^2$ | $R^3$ | $R^4$ | ΔΔE |
|---|---|---|---|---|---|
| 2 | $CH_3$ | H | H | $N(CH_3)C_2H_4OH$ | 0,18 |
| 3 | $OCH_3$ | H | H | $N(CH_3)C_2H_4OH$ | 0,22 |
| 4 | Cl | H | H | $N(CH_3)C_2H_4OH$ | 0,24 |
| 5 | $SO_2NH_2$ | H | H | $N(CH_3)C_2H_4OH$ | 0,34 |
| 6 | CN | H | H | $N(CH_3)C_2H_4OH$ | 0,40 |
| 7 | CN | H | CN | $N(CH_3)C_2H_4OH$ | 0,43 |
| 8 | CN | CN | H | $N(CH_3)C_2H_4OH$ | 0,54 |
| | | | | | |
| 9 | H | H | H | $N(C_2H_5)C_2H_4OH$ | 0,17 |
| 10 | $OCH_3$ | H | H | $N(C_2H_5)C_2H_4OH$ | 0,24 |
| 11 | Cl | H | H | $N(C_2H_5)C_2H_4OH$ | 0,28 |
| 12 | $CF_3$ | H | H | $N(C_2H_5)C_2H_4OH$ | 0,39 |
| 13 | $NO_2$ | H | H | $N(C_2H_5)C_2H_4OH$ | 0,53 |
| 14 | $NO_2$ | H | H | $OCH_3$ | 0,09 |
| | | | | | |
| 15 | $CH_3$ | H | H | $N(CH_3)_2$ | 0,17 |
| 16 | $OCH_3$ | H | H | $N(CH_3)_2$ | 0,19 |
| 17 | H | H | H | $N(CH_3)_2$ | 0,21 |
| 18 | Cl | H | H | $N(CH_3)_2$ | 0,24 |
| 19 | Br | H | H | $N(CH_3)_2$ | 0,28 |
| 20 | CN | H | H | $N(CH_3)_2$ | 0,48 |
| 21 | $NO_2$ | H | H | $N(CH_3)_2$ | 0,56 |

**Beispiel 22**

Der Farbstoff aus Beispiel 1 wird nach literaturbekanntem Verfahren in Methylenchlorid in Gegenwart von Pottasche mit Methacrylchlorid umgesetzt, der resultierende Ester durch Säulenchromatographie an Kieselgel gereinigt und nach dem in Beispiel 1 beschriebenen Verfahren der ΔΔE-Wert zu 0,48 bestimmt.

Setzt man an Stelle des Farbstoffs aus Beispiel 1 die Farbstoffe aus den Beispielen 4 oder 6 ein und verfährt im übrigen wie vorstehend angegeben, so erhält man ΔΔE-Werte von 0,27 bzw. 0,47.

Damit ist gezeigt, daß es nur auf die Gruppe A ankommt, die aber in weiten Grenzen substituiert sein darf und dadurch die ΔΔE-Werte variiert.

**Beispiel 23**

Verwendet man in dem Bestimmungsverfahren in Beispiel 1 an Stelle des dort eingesetzten Standards 1.3-Dinitrobenzol eine 1 molare Lösung des 4-Cyano-4'-hydroxybiphenyls, so beträgt der ΔΔE-Wert 0,37, setzt man an Stelle des Standards 1.3-Dinitrobenzols den N-(4-Cyanophenyl)-4-carbamidophenyl-2-oxyethylen-methacrylsäureester ein,

so beträgt der ΔΔE-Wert 0,47.

Damit ist gezeigt, daß auch der Standard austauschbar ist und im Einzelfalle an die Besonderheiten des Systems angepaßt werden kann.

**Beispiel 24**

Copolymere aus den Methacrylsäureestern der in den Beispielen 1 bis 10 genannten Farbstoffe mit äquimolaren Mengen von

a) N-(4-Cyanophenyl)-4'-carbamidophenyl)-2-oxyethylen-methacrylsäureester und

b) 4-Cyano-biphenyl-4'-oxyethylen-methacrylsäureester

werden in Chloroform mit AIBN als Radikalstarter polymerisiert. Aus den Copolymeren werden Meßproben auf 2x2 cm große Glasplättchen der Dicke 1.1 mm hergestellt, indem man sie in einem Spincoater (Bauart Süss RC 5) placiert und mit 0,2 ml einer Lösung von 150 g der nachfolgend angegebenen Polymere in 1 Liter absolutem Tetrahydrofuran bei 2000 U/min in 10 sec beschichtet. Die Schicht ist 0,9 μ dick, transparent und amorph. Zwischen gekreuzten Polarisatoren erscheint die Fläche gleichmäßig dunkel. Anzeichen für polarisierende Bereiche werden nicht beobachtet.

Die Meßplättchen werden mit einem Ar-Ionen Laser mit einer Leistung von 60 mW bei einer Wellenlänge von 514 nm belichtet, wobei sich eine Doppelbrechung aufbaut, die gemessen wird. Man erhält:

| **Farbstoffbeispiel** | **ΔΔE** | **Δn** | |
|---|---|---|---|
| | | Copolymer 24a | Copolymer 24b |
| 10 | 0,17 | | 0,011 |
| 2 | 0,18 | 0,021 | |
| 3 | 0,22 | 0,089 | 0,079 |
| 4 | 0,24 | | 0,019 |
| 6 | 0,40 | 0,063 | 0,074 |
| 7 | 0,43 | 0,038 | 0,077 |
| 8 | 0,54 | 0,024 | |
| 1 | 0,56 | 0,074 | 0,116 |

Das Beispiel zeigt, daß der ΔΔE-Wert unabhängig von der Wahl der Gruppe M die Vertreter mit einer hohen Doppelbrechungsänderung richtig voraussagt.

## Patentansprüche

**1.** Verfahren zur Auswahl von optisch isotropen Polymeren für die reversible, optische Datenspeicherung mit einer als Rückgrat wirkenden Hauptkette und davon abzweigenden kovalent gebundenen Seitengruppen der Formeln

$$-S^1-T^1-Q^1-A \qquad (I)$$

und

$$-S^2-T^2-Q^2-M \qquad (II)$$

worin

$S^1$, $S^2$ die Atome O, S oder den Rest $NR^0$ bedeuten,
$R^0$ Wasserstoff oder $C_1$-$C_4$-Alkyl bedeutet,

$T^1$, $T^2$ den Rest $(CH_2)_n$, der gegebenenfalls durch -O-, $-NR^0-$ oder $-OSiR^0{}_2O-$ unterbrochen sein kann und/oder gegebenenfalls durch Methyl oder Ethyl substituiert sein kann, bedeuten,

$Q^1$, $Q^2$ eine direkte Einfachbindung, -O-, -COO-, -OCO-, $-CONR^0-$, $-NR^0CO-$ oder $-NR^0-$ bedeuten

oder

$S^1T^1Q^1$ oder $S^2T^2Q^2$ eine bivalente Gruppe der Formel

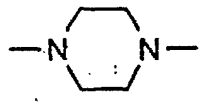

bedeuten,

A eine Einheit, die elektromagnetische Strahlung aufnehmen kann, bedeutet,

wobei für A die Formeln

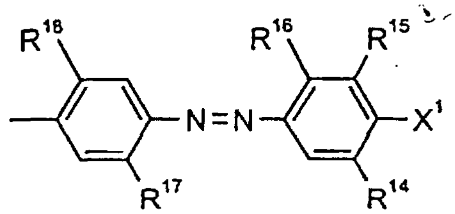

(XII)

worin

$R^{14}$ bis $R^{16}$ unabhängig voneinander $C_1$-$C_6$-Alkyl, Hydroxyl, $C_1$-$C_6$-Alkoxy, Phenoxy, $C_1$-$C_6$-Alkylthio, Phenylthio, Halogen, $CF_3$, $CCl_3$, $CBr_3$, Nitro, Cyan, $C_1$-$C_6$-Alkylsulfonyl, Phenylsulfonyl, $COOR^1$, Aminosulfonyl, $C_1$-$C_6$-Alkylaminosulfonyl, Phenylaminosulfonyl, Aminocarbonyl, $C_1$-$C_6$-Alkylaminocarbonyl, Phenylaminocarbonyl bedeuten,

$R^{17}$ Halogen, $C_1$-$C_6$-Alkyl, Hydroxyl, $C_1$-$C_6$-Alkoxy, Phenoxy, $C_1$-$C_4$-Acylamino, $C_1$-$C_4$-Alkylsulfonylamino bedeutet,

$R^{18}$ Halogen, $C_1$-$C_6$-Alkyl, Hydroxyl, $C_1$-$C_6$-Alkoxy, Phenoxy bedeutet,

$X^1$ Wasserstoff, Hydroxyl, Mercapto, $CF_3$, $CCl_3$, $CBr_3$, Halogen, Cyan, Nitro, $COOR^{19}$, $C_1$-$C_6$-Alkyl, $C_5$-$C_{12}$-Cycloalkyl, $C_1$-$C_{12}$-Alkoxy, $C_1$-$C_{12}$-Alkylthio, $C_6$-$C_{12}$-Aryl, $C_6$-$C_{12}$Aryloxy, $C_6$-$C_{12}$-Arylthio, $C_1$-$C_6$-Alkylsulfonyl, $C_6$-$C_{12}$-Arylsulfonyl, Ammosulfonyl, $C_1$-$C_6$-Alkylaminosulfonyl, Phenylaminosulfonyl, Aminocarbonyl, $C_1$-$C_6$-Alkylaminocarbonyl, Phenylaminocarbonyl, $NR^{19}$,$R^{20}$, NH-CO-$R^{19}$, NH-$SO_2$-$R^{19}$, NH-CO-$NR^{19}R^{20}$, NH-CO-O-$R^{19}$ oder $SO_2$-$CF_3$ bedeutet, worin $R^{19}$ und $R^{20}$ unabhängig voneinander für Wasserstoff, $C_1$-$C_4$-Alkyl oder Phenyl stehen, ausgenommen sind.

M eine mesogene, formanisotrope Einheit bedeutet

und

n eine ganze Zahl von 2 bis 12 bedeutet,

**dadurch gekennzeichnet, daß** A einen Extinktionsmodul ΔΔE von größer als 0,2 besitzt, gemessen an einer

Verbindung der Formel A-$Q^1$H oder A$Q^1T^1S^1$H durch 6 Einzelmessungen, nämlich:

A) A-$Q^1$H oder A$Q^1T^1S^1$H in $10^{-3}$ molarer Dioxan-Lösung,
B) Standard, bevorzugt 1,3 Dinitrobenzol, 1-molarer Konzentration im gleichen Lösungsmittel,
C) A-$Q^1$H oder A$Q^1T^1S^1$H und Standard in Konzentration wie in A) bzw. B) angegeben im gleichen Lösungsmittel

jeweils zweimal an der längerwelligen Kante der Absorptionskurve gemessen, und zwar einmal bei derjenigen Wellenlänge $\lambda$, bei der die Extinktion der Kurve c) 0,8 beträgt, und einmal bei der Wellenlänge $\lambda$ + 50 nm,

wobei man die drei Differenzen der Extinktionen $\Delta E = E_\lambda - E_{\lambda+50}$ für die Inhaltsstoffe A) bis C) bildet und also die drei Werte $\Delta E_A$ und $\Delta E_B$ und $\Delta E_C$ erhält, wobei dann der gesuchte Wert $\Delta\Delta E$ die Differenz $\Delta\Delta E = \Delta E_C - (\Delta E_B + \Delta E_A)$ ist.

2. Verwendung der Polymere ausgewählt gemäß Anspruch 1 zur Herstellung von mit Licht beschreibaren polymeren Filmen und optischen Bauelementen.

3. Polymere Filme und/oder optische Bauelemente, hergestellt aus Polymeren, ausgewählt gemäß Anspruch 1.

## Claims

1. Process for the selection of optically isotropic polymers for reversible optical data storage, which polymers have a main chain acting as the backbone and, branching therefrom, covalently bonded side groups of the formulae

$$-S^1-T^1-Q^1-A \qquad (I)$$

and

$$-S^2-T^2-Q^2-M \qquad (II)$$

wherein

$S^1$, $S^2$ represent the atoms O, S or the radical $NR^0$,

$R^0$ represents hydrogen or $C_1$-$C_4$-alkyl,

$T^1$, $T^2$ represent the radical $(CH_2)_n$, which may optionally be interrupted by -O-, -$NR^0$- or by -$OSiR^0_2O$- and/or may optionally be substituted by methyl or by ethyl,

$Q^1$, $Q^2$ represent a direct single bond, -O-, -COO-, -OCO-, -$CONR^0$-, -$NR^0CO$- or -$NR^0$-

or

$S^1T^1Q^1$ or $S^2T^2Q^2$ represents a divalent group of the formula

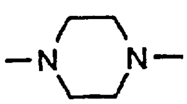 ,

A represents a unit which is able to absorb electromagnetic radiation,

with the following formulae being excluded for A:

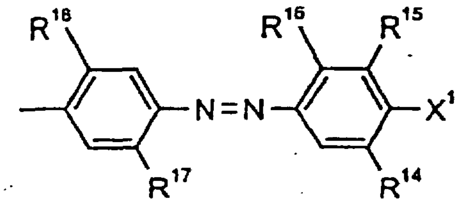

(XII)

wherein

$R^{14}$ to $R^{16}$ each independently of the others represents $C_1$-$C_6$-alkyl, hydroxyl, $C_1$-$C_6$-alkoxy, phenoxy, $C_1$-$C_6$-alkylthio, phenylthio, halogen, $CF_3$, $CCl_3$, $CBr_3$, nitro, cyano, $C_1$-$C_6$-alkylsulfonyl, phenylsulfonyl, $COOR^1$, aminosulfonyl, $C_1$-$C_6$-alkylaminosulfonyl, phenylaminosulfonyl, aminocarbonyl, $C_1$-$C_6$-alkylaminocarbonyl, phenylaminocarbonyl,

$R^{17}$ represents halogen, $C_1$-$C_6$-alkyl, hydroxyl, $C_1$-$C_6$-alkoxy, phenoxy, $C_1$-$C_4$-acylamino, $C_1$-$C_4$-alkylsulfonylamino,

$R^{18}$ represents halogen, $C_1$-$C_6$-alkyl, hydroxyl, $C_1$-$C_6$-alkoxy, phenoxy,

$X^1$ represents hydrogen, hydroxyl, mercapto, $CF_3$, $CCl_3$, $CBr_3$, halogen, cyano, nitro, $COOR^{19}$, $C_1$-$C_6$-alkyl, $C_5$-$C_{12}$-cycloalkyl, $C_1$-$C_{12}$-alkoxy, $C_1$-$C_{12}$-alkylthio, $C_6$-$C_{12}$-aryl, $C_6$-$C_{12}$-aryloxy, $C_6$-$C_{12}$-arylthio, $C_1$-$C_6$-alkylsulfonyl, $C_6$-$C_{12}$-arylsulfonyl, aminosulfonyl, $C_1$-$C_6$-alkylaminosulfonyl, phenylaminosulfonyl, aminocarbonyl, $C_1$-$C_6$-alkylaminocarbonyl, phenylaminocarbonyl, $NR^{19}$,$R^{20}$, NH-CO-$R^{19}$, NH-$SO_2$-$R^{19}$, NH-CO-$NR^{19}R^{20}$, NH-CO-O-$R^{19}$ or $SO_2$-$CF_3$, wherein $R^{19}$ and $R^{20}$ each independently of the other represents hydrogen, $C_1$-$C_4$-alkyl or phenyl,

M represents a mesogenic unit having anisotropy of form,

and

n represents an integer from 2 to 12,

which process is **characterised in that** A has an extinction modulus ΔΔE of greater than 0.2, measured on a compound of the formula A-$Q^1$H or $AQ^1T^1S^1H$ by 6 individual measurements, namely:

A) A-$Q^1$H or $AQ^1T^1S^1H$ in $10^{-3}$ molar dioxane solution,

B) standard, preferably 1,3-dinitrobenzene, 1-molar concentration in the same solvent,

C) A-$Q^1$H or $AQ^1T^1S^1H$ and standard in the concentration indicated in A) or B), respectively, in the same solvent,

each measured twice at the edge of the absorption curve of longer wavelength, namely once at the wavelength λ at which the extinction of the curve c) is 0.8 and once at the wavelength λ + 50 nm,
the three differences in the extinctions $\Delta E = E_\lambda - E_{\lambda+50}$ being formed for the substances A) to C) and the three values $\Delta E_A$ and $\Delta E_B$ and $\Delta E_C$ thus being obtained, the desired value ΔΔE then being the difference $\Delta\Delta E = \Delta E_C - (\Delta E_B + \Delta E_A)$.

**2.** Use of the polymers selected according to claim 1 in the production of polymeric films and optical components that are inscribable by means of light.

**3.** Polymeric films and/or optical components, produced from polymers selected according to claim 1.

## Revendications

**1.** Procédé pour le choix de polymères optiquement isotropes prévus pour l'enregistrement optique réversible de données, ayant une chaîne principale faisant fonction de squelette et, en ramifications, des groupes latéraux reliés par des liaisons covalentes et répondant aux formules

$$-S^1-T^1-Q^1-A \qquad (I)$$

et

$$-S^2-T^2-Q^2-M \qquad (II)$$

dans lesquelles,

| | |
|---|---|
| $S^1$, $S^2$ | représentent les atomes O, S ou le groupe $NR^0$, |
| $R^0$ | représente l'hydrogène ou un groupe alkyle en $C_1$-$C_4$, |
| $T^1$, $T^2$ | représentent le groupe $(CH_2)_n$ éventuellement interrompu par -O-, $-NR^0-$ ou $-OSiR^0_2O-$ et/ou qui peut éventuellement être substitué par des groupes méthyle ou éthyle, |
| $Q^1$, $Q^2$ | représentent une liaison directe simple, -O-, -COO-, -OCO-, $-CONR^0-$, $-NR^0CO-$ ou $-NR^0-$ ou bien |
| $S^1T^1Q^1$ ou $S^2T^2Q^2$ | représente un groupe bivalent de formule |

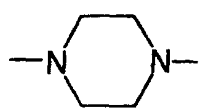

| | |
|---|---|
| A | représente un motif capable d'absorber un rayonnement électromagnétique, |

A étant choisi parmi les composés de formule

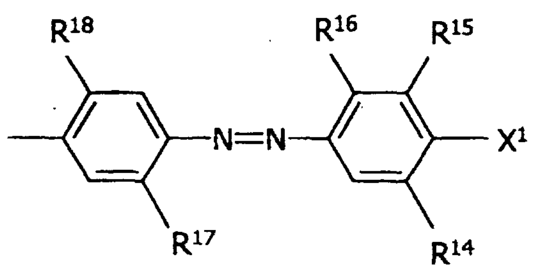

(XII)

dans laquelle

| | |
|---|---|
| $R^{14}$ à $R^{16}$ | représentent chacun, indépendamment les uns des autres, un groupe alkyle en $C_1$-$C_6$, hydroxy, alcoxy en $C_1$-$C_6$, phénoxy, alkylthio en $C_1$-$C_6$, phénylthio, un halogène, $CF_3$, $CCl_3$, $CBr_3$, nitro, cyano, alkylsulfonyle en $C_1$-$C_6$, phénylsulfonyle, $COOR^1$, aminosulfonyle, (alkyle en $C_1$-$C_6$)aminosulfonyle, phénylaminosulfonyle, aminocarbonyle, (alkyle en $C_1$-$C_6$)aminocarbonyle, phénylaminocarbonyle, |
| $R^{17}$ | représente un halogène, un groupe alkyle en $C_1$-$C_6$, hydroxy, alcoxy en $C_1$-$C_6$, phénoxy, acylamino en $C_1$-$C_4$, alkylsulfonylamino en $C_1$-$C_4$, |
| $R^{18}$ | représente un halogène, un groupe alkyle en $C_1$-$C_6$, hydroxy, alcoxy en $C_1$-$C_6$, phénoxy, |
| $X^1$ | représente l'hydrogène, un groupe hydroxy, mercapto, $CF_3$, $CCl_3$, $CBr_3$, un halogène, un groupe cyano, nitro, $COOR^{19}$, alkyle en $C_1$-$C_6$, cycloalkyle en $C_5$-$C_{12}$, alcoxy en $C_1$-$C_{12}$, alkylthio en $C_1$-$C_{12}$, aryle en $C_6$-$C_{12}$, aryloxy en $C_6$-$C_{12}$, arylthio en $C_6$-$C_{12}$, alkylsulfonyle en $C_1$-$C_6$, arylsulfonyle en $C_6$-$C_{12}$, aminosulfonyle, alkylaminosulfonyle $C_1$-$C_6$, phénylaminosulfonyle, aminocarbonyle, (alkyle |

en $C_1$-$C_6$)aminocarbonyle, phénylaminocarbonyle, $NR^{19}$,$R^{20}$, NH-CO-$R^{19}$, NH-$SO_2$-$R^{19}$, NH-CO-$NR^{19}R^{20}$, NH-CO-O-$R^{19}$ ou $SO_2$-$CF_3$, dans lesquels $R^{19}$ et $R^{20}$ représentent chacun, indépendamment l'un de l'autre, l'hydrogène, un groupe alkyle en $C_1$-$C_4$ ou phényle,

M représente un motif mésogène anisotrope de forme

et

n est un nombre entier allant de 2 à 12,

**caractérisé en ce que** A a un module d'extinction ΔΔE supérieur à 0,2, la mesure étant faite sur un composé de formule A-$Q^1$H ou $AQ^1T^1S^1$H en six mesures individuelles, à savoir :

A) sur une solution $10^{-3}$ molaire de A-$Q^1$H ou $AQ^1T^1S^1$H dans le dioxanne,
B) sur un étalon, de préférence le 1,3-dinitrobenzène, à une concentration molaire dans le même solvant,
C) sur A-$Q^1$H ou $AQ^1T^1S^1$H et l'étalon à une concentration identique à celle respectivement de A) ou B) et dans le même solvant, à deux reprises dans chaque cas sur le bord à plus grandes longueurs d'onde de la courbe d'absorption, à savoir une fois à la longueur d'onde λ à laquelle l'extinction de la courbe c) est de 0,8, et une fois à la longueur d'onde λ+50 nm,
d'où l'on calcule les trois différences des extinctions $\Delta E = E_{\lambda} - E_{\lambda+50}$ pour les constituants A) à C) et donc les trois valeurs $\Delta E_A$ et $\Delta E_B$ et $\Delta E_C$ puis la valeur recherchée ΔΔE qui est la différence $\Delta\Delta E = \Delta E_C - (\Delta E_B + \Delta E_A)$.

**2.** Utilisation des polymères choisis selon la revendication 1 pour la fabrication de pellicules polymères inscriptibles sous l'action de la lumière et d'éléments de construction optiques.

**3.** Pellicules de polymères et/ou éléments de construction optique fabriqués à partir de polymères choisis selon la revendication 1.